# EUROPEAN PATENT SPECIFICATION

(11) **EP 3 113 786 B1**
(45) Date of publication and mention of the grant of the patent: **19.02.2020**
(21) Application number: 15709139.8
(22) Date of filing: 04.03.2015
(51) Int. Cl.: A61K 38/10, A61K 38/19, A61P 29/00

(54) **ATTENUATION OF INTRAPULMONARY INFLAMMATION**
LINDERUNG VON INTRAPULMONALEN ENTZÜNDUNGEN
ATTÉNUATION D'UNE INFLAMMATION INTRAPULMONAIRE

(30) Priority: 04.03.2014 AT 501582014
(43) Date of publication of application: 11.01.2017
(73) Proprietor: APEPTICO Forschung und Entwicklung GmbH, 1150 Wien (AT)
(72) Inventor: FISCHER, Bernhard, A-1160 Wien (AT)
(74) Representative: Schwarz & Partner Patentanwälte OG
(86) International application number: PCT/EP2015/054493
(87) International publication number: WO 2015/132294

(56) References cited:
- EP-A1- 2 397 151
- EP-A1- 2 679 239
- EP-A1- 2 679 239
- WO-A1-90/06945
- WO-A1-2006/013183
- WO-A1-2010/099556
- WO-A1-2012/065201
- AT-A1- 509 267
- PARASTOO HAZEMI ET AL: "Essential structural features of TNF-alpha lectin-like domain derived peptides for activation of amiloride-sensitive sodium currecnt in A549 cells", JOURNAL OF MEDICINAL CHEMISTRY, AMERICAN CHEMICAL SOCIETY, vol. 53, no. 22, 27 October 2010 (2010-10-27), pages 8021-8029, XP002638326, DOI: 10.1021/JM100767P

## Description

The present invention relates to the attenuation of intrapulmonary inflammation by administration of specific compounds.

Sepsis is a potentially fatal whole-body inflammation caused by severe infection. Sepsis can continue even after the infection that caused it is gone. Severe sepsis may cause organ dysfunction, including lung dysfunction. (Levy, Mitchell M.; Fink, Mitchell P.; Marshall, John C.; Abraham, Edward; Angus, Derek; Cook, Deborah; Cohen, Jonathan; Opal, Steven M.; Vincent, Jean-Louis; Ramsay, Graham (2003). "2001 SCCM/ESICM/ACCP/ATS/SIS International Sepsis Definitions Conference". Critical Care Medicine 31 (4): 1250-6.)

Sepsis is caused by the immune system's response to a serious infection, most commonly bacteria, but also fungi, viruses, and parasites in the blood, urinary tract, lungs, skin, or other tissues. Sepsis can be thought of as falling within a continuum from infection to multiple organ dysfunction syndrome. (Annane D, Bellissant E, Cavaillon JM (2005). "Septic shock". Lancet 365 (9453): 63-78.)

Common symptoms of sepsis include those related to a specific infection, but usually accompanied by high fevers, hot, flushed skin, elevated heart rate, hyperventilation, altered mental status, swelling, and low blood pressure
Sepsis is usually treated with intravenous fluids and antibiotics. If fluid replacement is not sufficient to maintain blood pressure, vasopressors can be used. Mechanical ventilation and dialysis may be needed to support the function of the lungs and kidneys, respectively. The use of corticosteroids is controversial. Activated drotrecogin alfa (recombinant activated protein C), originally marketed for severe sepsis, has not been found to be helpful, and has recently been withdrawn from sale.

Sepsis and pulmonary inflammation can be determined by the degree of accumulation inflammation markers and modulators (inflammatory cytokines), such as is tumor-necrosis-factor-α (TNF-α), immune cells and alveolar macrophages, in the lung fluid.

Lipopolysaccharide (LPS) becomes present as glycolipids of gram-negative bacteria in systemic bacteremia and can trigger inflammatory response to the point of septic shock and cardio-circulatory failure. Systemic effects of LPS include hemodynamic deterioration along with increased pulmonary arterial pressure and acute leucopenia.

It was now surprisingly found that certain peptides are active under conditions of systemic sepsis or inflammatory response. Repetitive inhalation of certain peptides led to a significantly lower intrapulmonary expression of inflammatory cytokines (IL-6, TNF-α) and enzymes (COX-2) in a primarily systemic sepsis model. It was also surprisingly found that the repetitive application of such peptides attenuates intrapulmonary inflammation despite a systemic inflammatory response induced by LPS infusion.

The present invention is defined by the claims. In one aspect the present disclosure provides a cyclized compound of the amino acid sequence of formula

X₁-GQRETPEGAEAKPWY-X₂ I

(SEQ ID NO:9) wherein
X₁ comprises an amino acid (sequence) with 1 to 4, in particular 1 to 3 members, comprising natural or unnatural amino acids, in particular selected from the amino acid (sequence) C, KSP, K, ornithin, 4-amino butanoic acid, β-alanine, and
X₂ comprises one amino acid, selected from natural amino acids, in particular selected from the group C, D, G and E,
and wherein
X₁ comprises the N-terminal amino acid at ist first left position and X₂ comprises the C-terminal amino acid at its last right position,
for use in the treatment of inflammation.

A cyclized compound of formula I for use in inflammation is designated herein also as a "compound of (according to) the present invention". The use of a cyclized compound of formula I in inflammation is herein also designated as a "use of (according to) the present invention".

In a compound of the present invention cyclization is performed by reaction of a reactive chemical group in one of the amino acids of X₁, preferably in the terminal amino acid of X₁, and a reactive chemical group of the amino acid X₂, e.g. by reaction of reactive groups of the C-terminal amino acid and the N-terminal amino acid.

"Inflammation" as in accordance with the present invention herein includes intrapulmonary inflammation, sepsis, systemic and organ inflammation.

In one preferred aspect the present invention provides the use of the present invention for the treatment of intrapulmonary inflammation, in another aspect for the treatment of sepsis, in a further aspect for the treatment of systemic inflammation and in another aspect for the treatment of organ inflammation.

Treatment as used herein includes treatment and prevention.

Natural amino acids useful in an amino acid sequence in the present disclosure are known and comprise e.g. G, A, V, L, I, M, P, F, W S, T, N, Q, C, U, Y, D, E, H, K, R.

Unnatural amino acids useful in an amino acid sequence in a method of the present disclosure comprise
- amino acids which have the principal structure of natural amino acids, but which are other than alpha amino acids,
- natural amino acids in the D-form, namely other than in the natural L-form, i.e. natural amino acids, wherein the alkyl group is not in the L-configuration, but in the D-configuration,
- unnatural amino acids comprising from 2 to 12, such as from 2 to 6 carbon atoms, at least one amino group, e.g. one or two, and at least one carboxy group, e.g. one or two, e.g. optionally beside substituents which are present also in natural amino acids, such as e.g. OH, -CONH₂,-NH-C(=NH₂)NH₂, SH, (C₁₋₄)alkyl-S-, phenyl, heterocyclyl, e.g. heterocyclyl comprising 5 or 6 ring members and comprising at least on heteroatom selected from N, O, S, preferably N, e.g. one or two N, optionally anellated with another ring, such as phenyl, e.g. including prolinyl, indolyl, imidazolyl;

In one specific aspect, unnatural amino acids in an amino acid sequence in a method of the present disclosure include ortithin, 4-aminobutyric acid, β-alanine, 7-amino-heptanoic acid, 6-amino-hexanoic acid.

In another aspect a cyclized compound of the amino acid sequence of formula I includes
- a sequence SEQ ID NO: 1
   *Cyclo*(CGQRETPEGAEAKPWYC)
   wherein both terminal cysteine residues form a disulphide bridge;
- a sequence SEQ ID NO:2
   *Cyclo*(KSPGQRETPEGAEAKPWYE) wherein an amide bond is formed between the amino group attached to the ε-carbon atom of the N-terminal lysine residue and the side chain carboxyl group attached to the γ-carbon of the C-terminal glutamic acid residue;
- a sequence SEQ ID NO:3
   *Cyclo*(KGQRETPEGAEAKPWYG)
   wherein an amide bond is formed between the amino group attached to the ε-carbon atom of the side chain of the N-terminal lysine residue and the carboxyl group of the C-terminal glycine residue;
- a sequence SEQ ID NO:4
   *Cyclo*(ornithine-GQRETPEGAEAKPWYG)
   wherein an amide bond is formed between the amino group attached to the δ-carbon of the side chain of the N-terminal ornithine residue and the carboxyl group of the C-terminal glycine residue;
- a sequence SEQ ID NO:5
   Cyclo(4-aminobutanoic acid-GQRETPEGAEAKPWYD)
   wherein an amide bond is formed between the amino group of the N-terminal 4-aminobutanoic acid residue and the side chain carboxyl group attached to the β-carbon of the C-terminal aspartic acid residue;
- a sequence SEQ ID NO:6
   Cyclo(β-alanine-GQRETPEGAEAKPWYE)
   wherein an amide bond is formed between the amino group of the N-terminal β-alanine (3-aminopropanoic acid) residue and the side chain carboxyl group attached to the γ-carbon of the C-terminal glutamic acid residue,
- a sequence SEQ ID NO: 7
   {[7-amino-heptanoic acid-GQRETPEGAEAKPWY] (cyclo 1-16)},
   the amino acids are peptidically linked from the C-terminal amino acid tyrosine to the N-terminal amino acid glycine, whereas the C-terminal amino acid tyrosine is linked to the N-terminal amino acid glycine via an amide bond between the nitrogen of the amino group of the N-terminal glycine and the carbon C1 of the carboxyl group of the 7-amino-heptanoic acid, on the one hand, and via an amide bond between the nitrogen of the amino group of the 7-amino-heptanoic acid and the carbon of the carboxyl group of the C-terminal tyrosine, on the other hand, so that the compound has neither an N-terminal amino group, nor a C-terminal carboxyl group, and
- a sequence SEQ ID NO: 8
   {[6-amino-hexanoic acid-GQRETPEGAEAKPWYG] (cyclo 1-17)}
   the amino acids are peptidically linked from the C-terminal amino acid glycine to the N-terminal amino acid glycine, whereas the C-terminal amino acid glycine is linked to the N-terminal amino acid glycine via an amide bond between the nitrogen of the amino group of the N-terminal glycine and the carbon C1 of the carboxyl group of the 6-amino-hexanoic acid, on the one hand, and via an amide bond between the nitrogen of the amino group of the 6-amino-hexanoic acid and the carbon of the carboxyl group of the C-terminal glycine, on the other hand, so that the compound has neither an N-terminal amino group, nor a C-terminal carboxyl group.

An embodiment of the present invention is a cyclized compound of formula I of the amino acid sequence SEQ ID NO:5, namely Cyclo(4-aminobutanoic acid-GQRETPEGAEAKPWYD), wherein an amide bond is formed between the amino group of the N-terminal 4-aminobutanoic acid residue and the side chain carboxyl group attached to the β-carbon of the C-terminal aspartic acid residue;

A compound of the present invention includes a compound in any form, e.g. in free form and in the form a salt, e.g. in biological environment a cyclized compound of the present invention normally is in the form of a salt.

In another aspect of the present invention provides a compound of formula I in the form of a salt.

Such salts include preferably pharmaceutically acceptable salts, although pharmaceutically unacceptable salts are included, e.g. for preparation / isolation / purification purposes.

In biological environment a salt of a cyclized compound of the present invention is normally a hydrochloride.

A cyclized compound of the present invention in free form may be converted into a corresponding compound in the form of a salt; and vice versa.

A compound of the present invention may exist in the form of isomers and mixtures thereof; e.g. optical isomers. A compound of the present invention may e.g. contain asymmetric carbon atoms and may thus exist in the form of enatiomers or diastereoisomers and mixtures thereof, e.g. racemates. A compound of the present invention may be present in the (R)-, (S)- or (R,S)-configuration preferably in the (R)- or (S)-configuration regarding each of the substituents at such asymmetric carbon atoms in a compound of the present invention. Isomeric mixtures may be separated as appropriate, e.g. according, e.g. analogously, to a method as conventional, to obtain pure isomers. The present invention includes a compound of the present invention in any isomeric form and in any isomeric mixture. In case of natural amino acids the configuration of substituents is as in natural amino acids.

A compound of the present invention may be prepared as appropriate, e.g. according, e.g. analogously, to a method as conventional, e.g. or as specified herein, e.g. by solid-phase peptide synthesis, optionally according to the fluorenylmethoxycarbonyl/t-butyl protection strategy on 2-chlorotritylchloride resin using appropriate coupling agents, such as diisopropyl carbodiimide and/or N-hydroxybenzotriazole and approipriate solvent, e.g. N,N- dimethylformamide. Protected amino acids may be coupled in succession to the peptide chain, starting with the C-terminal amino acid. Deprotection from fluorenylmethoxycarbonyl-protected groups may be carried out with a base, e.g. piperidine, such as 20% piperidine in an appropriate solvent, such as N-N-dimethyl formamide. The cleavage of the completed, optionally (partially) protected peptide from the resin may be carried out as appropriate, e.g. with an acid, such as acetic acid in appropriate solvent, e.g. halogenated hydrocarbon, such as CH₂Cl₂, e.g. in a 1:1 mixture of acetic acid and CH₂Cl₂.

In the case of cysteine-containing peptides, after cleavage from the resin, side-chain deprotection may be carried out, if desired, e.g. with a strong acid, such as trifluoroacetic acid (TFA), e.g. 95% TFA / 5% H₂O. Cyclization to obtain a disulfide bond may be carried out by oxidation of terminal cysteine residues, e.g. achievable by aeration of the crude linear peptide at pH 8.5 for 90 hours. Crude peptide product obtained may be purified, e.g. by chromatography, e.g. by reverse phase medium pressure liquid chromatography (RP-MPLC) on an appropriate column, such as RP-C18-silica gel column, conventiently using an eluent gradient, such as a gradient of 5% - 40% acetonitrile. A trifluoracetate counter-ion may be replaced, e.g. by acetate, e.g. over a column, such as over a Lewatit MP64 column (acetate form). Following a final wash in water, the purified peptide as acetate salt may be lyophilized and may b e obtained in the form of a light coloured, e.g. white powder.

In the case of cysteine-free peptides, the cyclization step may be carried out as appropriate, e.g. still on the partially-protected linear peptide, following the cleavage from the resin. After selective cyclization of the cysteine-free peptides, side-chain deprotection in TFA, if necessary, may be carried. A purification step may be carried out, e.g. via chromatography, e.g. by preparative RP-MPLC. From the peptide thus obtained replacement of the trifluoroacetate ion by acetate may be carried out, e.g. as described above. Lyophilization of the acetate form of the peptide may also be carried out, e.g. as for cysteine-containing peptides.

The molecular masses of peptides obtained may be confirmed by electrospray ionisation mass spectrometry or MALDI-TOF-MS. Purity may be determined, e.g. by analytical high performance liquid chromatography.

Such peptides and their preparation are e,g, described in WO 2011/085423.

The compounds of the present disclosure, e.g. including a compound of formula I, exhibit interesting pharmacological activity and are therefore useful as pharmaceuticals. E.g., study results as indicated below demonstrated that upon application of a compound of the present invention the intrapulmonary expression of inflammatory marker genes attenuate. These findings provide for the first time an anti-inflammatory effect in clinically relevant in vivo lung injury.

A compound of the present invention may be used as a pharmaceutical for inflammation treatment in the form of a pharmaceutical composition.

In another aspect the present disclosure provides a pharmaceutical composition for use in the treatment of inflammation, comprising a compound of the present invention,
and
a method of treatment of inflammation comprising administering an effective amount of a compound of the present invention to a mammal in need of such treatment.

For inflammation treatment with a compound of the present invention, the appropriate dosage will, of course, vary depending upon, for example, the chemical nature and the pharmacokinetic data of a compound of the present invention used, the individual host, e.g. the body weight, the age and the individual condition of a subject in need of such treatment, the mode of administration and the nature and severity of the conditions being treated.

However, in general, for satisfactory results in larger mammals, for example humans, an indicated daily dosage includes a range
- from about 0.0001 g to about 1.5 g, such as 0.001 g to 1.5 g;
- from about 0.001 mg/kg body weight to about 20 mg/kg body weight, such as 0.01 mg/kg body weight to 20 mg/kg body weight,
for example administered in divided doses up to four times a day.

Usually, children may receive half of the adult dose.

A compound of the present invention may be administered as appropriate.

A compound of the present invention may be administered by any conventional route, for example enterally, e.g. including nasal, buccal, rectal, oral administration; parenterally, e.g. including intravenous, intraarterial, intramuscular, intracardiac, subcutanous, intraosseous infusion, transdermal (diffusion through the intact skin), transmucosal (diffusion through a mucous membrane), inhalative administration, e.g. oral inhalation as aerosol;
e.g. in form of coated or uncoated tablets, capsules, (injectable) solutions, solid solutions, suspensions, dispersions, solid dispersions; e.g. in the form of ampoules, vials, in the form of creams, gels, pastes, inhaler powder, foams, tinctures, lip sticks, drops, sprays, or in the form of suppositories.

The compounds of the present invention may be administered in the form of a pharmaceutically acceptable salt, or in free form; optionally in the form of a solvate. A compound of the present invention in the form of a salt and/or in the form of a solvate exhibits the same order of activity as a compound of the present invention in free form.

It was surprisingly found that admistration of a cyclized compound of the present invention at best may be performed by inhalative administration.

Preferably a compound of the present invention is administered by inhalation, e.g. in the form of an aeorosol, either an aqueous solution, or a lyophilisate of a compound of the present invention, re-dissolved in water, is subjected to inhalation. Surprsingly it was found that the aqueous solution of a compound of the present disclosure comprising the invention, e.g. of (one of) the amino acid sequences SEQ ID NO:1 to SEQ ID NO:9 is also stable for a rather long time, even without addition of stabilizers and/or auxiliaries which usually are used. It was also found that the size of the vaporized droplets for inhalation comprising a dissolved compound of the present invention also may have an advantageous influence. E.g. in a preferred embodiment the droplet size of (most of) the atomized droplets does not exceed 5 µm (upper limit), in order to obtain a particularly successfull result. The appropriate lower limit of the droplet size is dependent only from the feasibility of the droplets.

For inhalative administration firstly a cyclized compound of the present disclosure comprising the invention, e.g. of (one of) the amino acid sequences SEQ ID NO:1 to SEQ ID NO:9 is dissolved in water, in order to obtain an aqueous solution and the solution obtained is optionally filtered, e.g. in order to remove impurities. The filtrate obtained is optionally lyophilized, e.g. for the case that a storage form is desired. Surprisingly it has been found that a lyophilized compound of the present invention thus obtained is stable for a long period. Stability of the lyophilisates was determined after up to 24 months at 2-8°C and up to 6 months at 25°C at 60% relative humidity. Fot that ususal laboratory analytical methods were used, e.g. visual inspection and reversed HPLC. After a storage of 24 months at 2-8°C also the die biological activity via Patch Clamp experiments was determined. The lyphilisates turned out to be stable under the conditions described, the appearance did not change, the content of the cyclized peptide of formuila I and purity showed only small variances, if even. Also the biological activity remained practically unchanged.

A compound of the present invention may be used for any method or use as described herein alone or in combination with one or more, at least one, other, second drug substance.

Combinations include fixed combinations, in which a compound of the present invention and at least one second drug substance are in the same formulation; kits, in which a compound of the present invention and at least one second drug substance in separate formulations are provided in the same package, e.g. with instruction for co-administration; and free combinations in which a compound of the present invention and at least one second drug substance are packaged separately, but instruction for concomitant or sequential administration are given.

Treatment with combinations according to the present invention may provide improvements compared with single treatment.

Pharmaceutical compositions according to the present invention may be manufactured according, e.g. analogously, to a method as conventional, e.g. by mixing, granulating, coating, dissolving or lyophilizing processes. Unit dosage forms may contain, for example, from about 0.1 mg to about 1500 mg, such as 1 mg to about 1000 mg.

Pharmaceutical compositions comprising a combination of the present invention and pharmaceutical compositions comprising a second drug as described herein, may be provided as appropriate, e.g. according, e.g. analogously, to a method as conventional, or as described herein for a pharmaceutical composition of the present invention.

By the term "second drug substance" is meant a chemotherapeutic drug, especially any chemotherapeutic agent other than a compound of the present invention, such as a compound of formula I.

For characterization of the effects of a compound of the present invention on inflammation, such as intrapulmonary inflammation, a porcine model of lipopolysaccharide (LPS)-induced sepsis was examined. As the active compound a compound of formula I of the amino acid sequence SEQ ID NO.5.

### Methods

Following animal care committee approval (Landesuntersuchungsamt Rheinland-Pfalz, Koblenz, Germany; approval number 23 177-07/G12-1-058) 18 juvenile pigs (weight 25-27 kg) were examined in a randomized, investigator-blinded setting.

### Anesthesia and Instrumentation

After sedation with intramuscular injection of ketamine (8 mg kg⁻¹) and midazolam (0.2 mg kg⁻¹) and preparation of vascular access, anesthesia was induced and maintained by intravenous propofol and fentanyl administration (8-12 mg kg⁻¹ h⁻¹ / 0.1-0.2 mg h⁻¹). A single dose of atracurium (0.5 mg kg⁻¹) was applied to facilitate orotracheal intubation. Ventilation (Respirator: AVEA®, CareFusion, USA) was started in pressure-controlled mode with a tidal volume of (Vₜ) of 8 mL kg⁻¹, positive end-expiratory pressure (PEEP) of 5 cmH₂O, FiO₂ of 0.3-0.4 and a variable respiratory rate to maintain normocapnia. A balanced saline solution (Sterofundin iso, B. Braun, Germany) was continuously infused at a rate of 10 mL kg⁻¹ h⁻¹. Vascular catheters were placed ultrasound-guided in Seldinger's technique and under sterile conditions: an arterial line, a pulse contour cardiac output catheter (PiCCO, Pulsion Medical Systems, Germany) and central venous line were inserted via femoral vein access. A 7.5-French introducer for a pulmonary artery catheter was placed via the right internal jugular vein. Ventilatory and extended hemodynamic parameters were recorded continuously (Datex S/5, GE Healthcare, Germany). Body temperature was measured by a rectal probe and normothermia was maintained by body surface warming.

### Experimental protocol

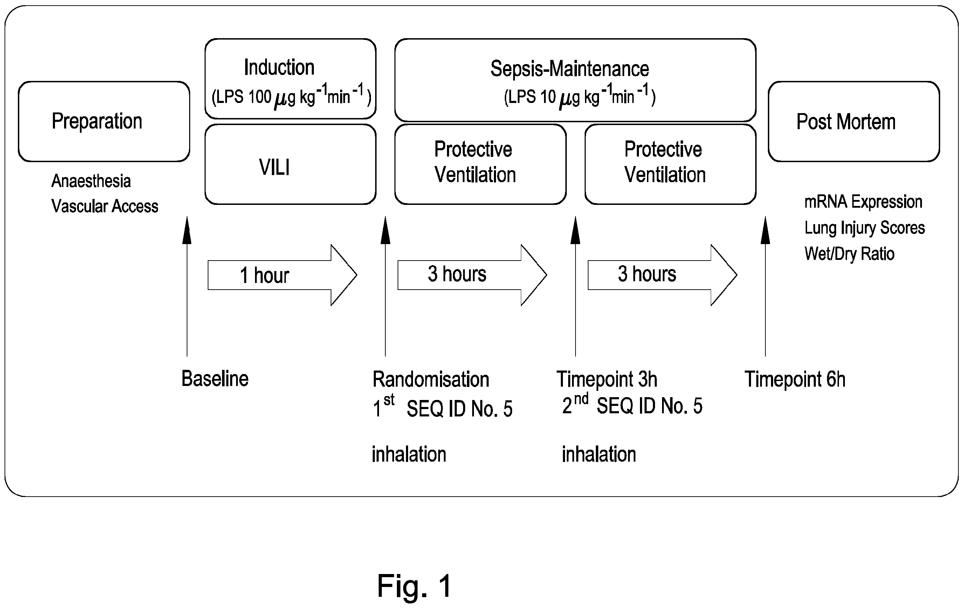

Following instrumentation baseline parameters were assessed at healthy state. Fig.1 summarizes the experimental protocol: systemic inflammation was induced by continuous LPS infusion (Escherichia coli serotype O111:B4, Sigma-Aldrich, Switzerland) for one hour at 100 µg kg⁻¹ h⁻¹, followed by 10 µg kg⁻¹ h⁻¹ for the entire experiment. Initial high-dose infusion was combined with a non-protective ventilation setting (Vₜ 25 mL kg⁻¹, zero PEEP, FiO₂ 1.0) to add a VILI component. Afterwards the ventilation mode was switched to a more lung protective setting: Vₜ of 8 mL kg⁻¹, PEEP 5 cm H₂O, FiO₂ of 0.4-0.5, and a variable respiratory rate to maintain a pH > 7.2. The animals were monitored over six hours after sepsis induction. During the induction phase a non-participant randomized the animals into two groups and prepared the peptide solution as previously described (Hartmann EK et al, Acta anaesthesiologica Scandinavica 2013, 57(3):334-341) for blinded endotracheal inhalation.

In the present study 2 groups were investigated:
Group (1) animals, to which 1 mg kg⁻¹ of a compound of formula I of the amino acid sequence SEQ ID NO:5 was administered at zero and three hours;
Group (2) animals, as the control group (CTRL), to which a vehicle solution at zero and three hours was administered.

To maintain hemodynamic stability (mean arterial pressure > 60 mmHg) additional fluid boli were administered (150 ml of balanced saline or hydroxyethyl starch once every hour). Persisting instability was treated by continuous central venous noradrenaline infusion. At the end of the experiments the animals were killed in deep general anesthesia by intravenous injection of propofol (200 mg) and potassium chloride (40 mval).

### Hematological parameters

Blood gas values were obtained using a Rapidlab 248 device (Bayer Healthcare, Germany). Hematological parameters were sampled during baseline, sepsis induction and after three and six hours. Lactate plasma levels, leucocyte and platelet counts were analyzed by the Institute of Laboratory Medicine, Medical Center of the Johannes Gutenberg-University. The plasma levels of IL-6 and TNF-α were determined by quantifying enzyme linked immunosorbent assays according to the manufacturer's instructions (Porcine IL-6 Quantikine ELISA, Porcine TNF-a Quantikine ELISA, R&D Systems, Germany).

### Histopathological and lung water content assessment

The lungs were removed en-bloc after thoracotomy. A macroscopic lung injury score was assessed as previously described in detail (Lim CM et al, Lung 2003, 181(1):23-34). Four ventral and dorsal segments (each upper/lower right, upper/lower left) of the lung surface were examined for hemorrhage and congestion (2 points > 50%, 1 point for < 50%, 0 points for no or minimal changes). The right lung was fixed in 10% buffered formalin. Representative tissue samples were paraffin embedded and cut for hematoxylin and eosin staining. A blinded investigator under supervision of a senior pathologist performed the histopathological assessment. In different lung regions (non-dependent periphery and bronchial, dependent periphery and bronchial) morphological changes were rated for seven criteria (alveolar edema, interstitial edema, hemorrhage, inflammatory infiltration, epithelial damage, microatelectasis and overdistension). The severity of each parameter ranged from 0 (no occurrence) to 5 points (complete field). For every lung region we used the mean value of four non-overlapping microscopy fields. The sum of the regional scores in all lung regions adds to a maximum injury score of 140 points (7 parameters x 5 maximum points per parameter from 4 lung regions). Additionally the regional distribution of each parameter was assessed in the dependent versus non-dependent lung regions. Similar scoring procedures were described previously (Spieth PMet al, Intensive Care Med 2007, 33(2):308-314; Wang HM et al, Eur Surg Res 2010, 45(3-4): 121-133).The left lung was weighted immediately after removal and dried afterwards at 60°C for 72 hours to determine the dry weight and wet to dry ratio (W/D).

### Gene expression analysis

To determine intrapulmonary inflammation mRNA levels of pro-inflammatory cytokines interleukin-1β (IL-1β), interleukin -6 (IL-6), TNF-α, and enzymes prostaglandin G/H synthase-2 (COX-2) and inducible nitric oxide synthase (iNOS) were quantified. Amphiregulin and tenascin-c expression levels were examined as surrogates of mechanical stress and remodeling. Four representative samples from the left lung (upper/lower lobe, each dependent/non-dependent) were collected, snap frozen in liquid nitrogen and stored at - 80°C. RNA extraction and quantification procedure by real-time polymerase chain reaction (Lightcycler 480 PCR System, Roche Applied Science, Germany) was conducted as previously described in detail.[17-19] mRNA expression data were normalized against peptidylprolyl isomerase A (PPIA) as control gene.

### Statistical analysis

Data are expressed as median and interquartile range (IQR) respectively box-plots. Intergroup comparisons were tested with the Mann-Whitney-U-Test. If multiple testing was performed, P values were adjusted by the Bonferroni correction. Intragroup time courses of repetitively measured parameters were analyzed by Friedman ANOVA on ranks and post-hoc Student-Newman-Keuls-Test. P values below 0.05 were regarded as significant.

Physiological data (ventilator and hemodynamic data) as set out in Table 1 were analyzed in explorative manner only. The statistical software SigmaPlot 11.0 (Systat Inc., USA) was used.

In Table 1 ventilator and hemodynamic data are set out .Data are presented as median (IQR), no relevant intergroup differences. Vₜ: tidal volume; P_{endinsp}: end-inspiratory pressure; PEEP: positive end-expiratory pressure; RR: respiratory rate; FiO₂: fraction of inspired oxygen; I:E: inspiration to expiration quotient; R_{aw}: airway resistance; EVLW: extravascular lung water content; PaCO₂: arterial partial pressure of carbon dioxide; MAP: mean arterial pressure; CO: cardiac output; CVP: central venous pressure; MPAP: mean pulmonary arterial pressure; NA: noradrenaline dosage.

### Results

### Physiological data

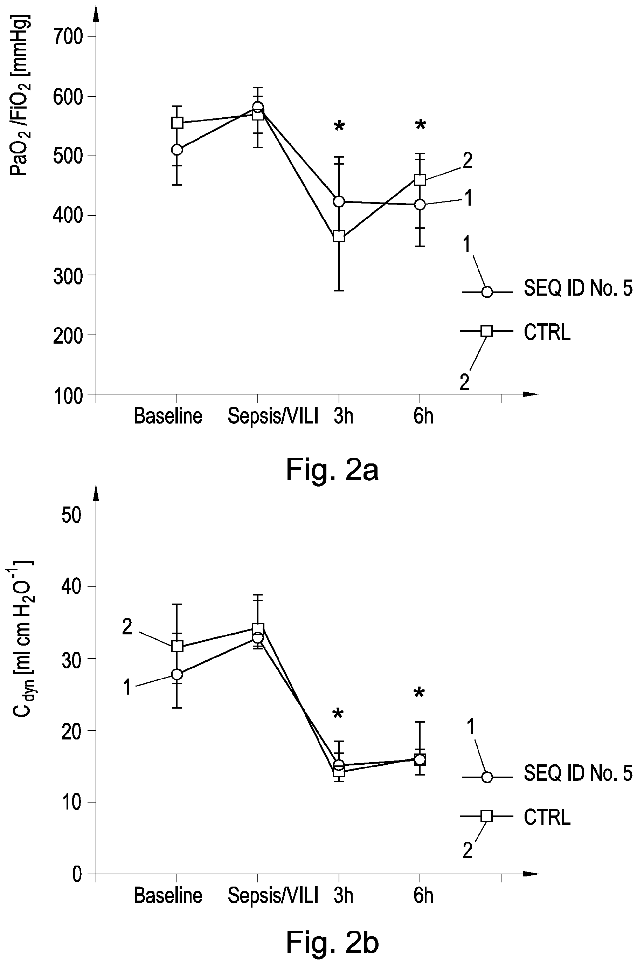

Table 1 summarizes the time charts of hemodynamic and respiratory parameters. During sepsis and ventilation the quotient of arterial partial pressure of oxygen and FiO₂ (PaO₂/FiO₂) did not decrease. Afterwards PaO₂/FiO₂ and dynamic lung compliance (C_{dyn}) significantly decreased within three hours in both groups and persisted without recovery (Fig. 2a and 2b). The two groups showed no significant differences. Hemodynamics were stable during baseline and sepsis/VILI induction, while over six hours continuous noradrenaline infusion was required in both groups in similar dosages.

### Systemic and pulmonary inflammatory response

The LPS infusion led to a sustained and persisting systemic leukopenia. This was accompanied by decreases in platelet count and rising lactate levels. Plasma levels of IL-6 and TNF-α increased significantly in both groups with a peak within three hours (Fig. 3a, 3b, 3c, 3d). Intrapulmonary mRNA quantification yielded significantly lower overall expression of COX-2 (p=0.003), TNF-α (p=0.041) and IL-6 (p=0.043) following inhalation of a compound of formula I of the amino acid sequence SEQ ID NO:5, with less differences in IL-1β and iNOS expressions (Fig. 4a, 4b, 4c, 4d; and Fig. 5a, 5b, 5c and 5d). Furthermore a decreased tenascin-c expression (p=0.015) was detected. No relevant locoregional variations were detected.

**Table 1 summarizes physiological data (ventilator and hemodynamic data), in particular the time charts of hemodynamic and respiratory parameters during sepsis and ventilation**

| | SEQ ID No:5 | | | | CTRL | | | |
|---|---|---|---|---|---|---|---|---|
| Parameter | Baseline | Sepsis/VILI | 3h | 6h | Baseline | Sepsis/VILI | 3h | 6h |
| Ventilation | | | | | | | | |
| Vₜ (mL kg⁻¹) | 8,4 (0,6) | 25,6 (1,7) | 8,5 (0,6) | 8,6 (0,4) | 8,3 (0,4) | 25,8 (0,4) | 8,3 (0,5) | 8,3 (0,7) |
| P_{endinsp} (cmH₂O) | 14 (1) | 22 (3) | 22 (2) | 19 (3) | 13 (2) | 21 (3) | 22 (3) | 19 (4) |
| RR (min⁻¹) | 29 (17) | 9 (2) | 34 (8) | 32 (8) | 35 (7) | 9 (2) | 33 (9) | 33 (12) |
| PEEP (cmH₂O) | 6 (1) | 1 (0,2) | 6 (1) | 5 (1) | 6 (1) | 1 (0,3) | 5 (1) | 5 (3) |
| Fio₂ | 0,4 | 1,0 | 0,4 | 0,4 | 0,4 | 1,0 | 0,4 | 0,4 |
| I:E | 1:2 | 1:2 | 1:2 | 1:2 | 1:2 | 1:2 | 1:2 | 1:2 |
| R_{aw} (cmH₂OL⁻¹s⁻¹) | 10 (2) | 12 (5) | 11 (2) | 11 (2) | 10 (2) | 12 (2) | 10 (2) | 8 (4) |
| EVLW (ml kg⁻¹) | 10 (2) | 11 (2) | 12 (2) | 13 (4) | 10 (1) | 13 (2) | 13 (3) | 14 (1) |
| PaCO₂ (mmHg) | 45 (6) | 35 (85) | 43 (6) | 43 (5) | 44 (3) | 36 (5) | 45 (3) | 41 (6) |

| Hemodynamics | | | | | | | | |
|---|---|---|---|---|---|---|---|---|
| MAP (mmHg) | 90 (14) | 100 (17) | 61 (14) | 66 (15) | 100 (21) | 111 (18) | 65 (24) | 60 (12) |
| CO (Lmin⁻¹) | 4,5 (0,4) | 4,5 (0,6) | 3,2 (0,9) | 3,7 (1,0) | 4,5 (0,6) | 4,2 (0,9) | 3,1 (0,2) | 3,4 (0,9) |
| CVP (mmHg) | 11 (4) | 10 (3) | 11 (4) | 13 (3) | 11 (2) | 11 (3) | 13 (2) | 14 (3) |
| MPAP (mmHg) | 22 (2) | 23 (3) | 33 (4) | 30 (12) | 21 (4) | 25 (4) | 39 (5) | 30 (12) |
| NA (*µ*g kg⁻¹min⁻¹) | 0 | 0 | 0,2 (1,3) | 0,8 (0,4) | 0 | 0 | 0,4 (0,7) | 0,9 (3,4) |

### Pathologic parameters

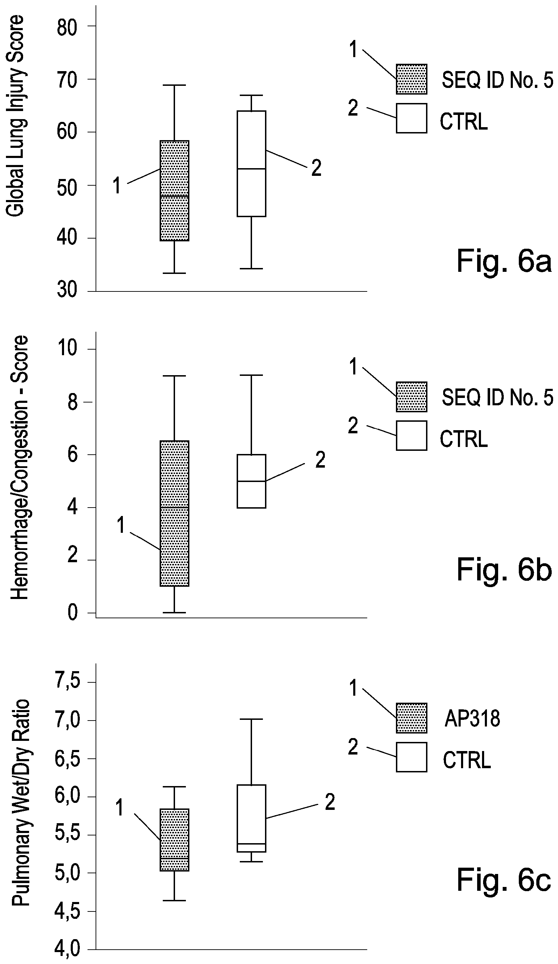

Post-mortem macroscopic and histologic evaluations yielded the presence of a sustained lung injury in both groups. Group (1) animals show a trend towards a less pronounced damage as well as higher W/D ratio (Fig. 6a, 6b, 6c). The most relevant features of the histopathological scoring were inflammatory infiltration as well as development of overdistended areas and atelectasis with edema formation playing a minor role. The CTRL animal Group (2) featured a higher grade of hemorrhage as set out in Table 2. No relevant differences were detected regarding the ventral to dorsal distribution.

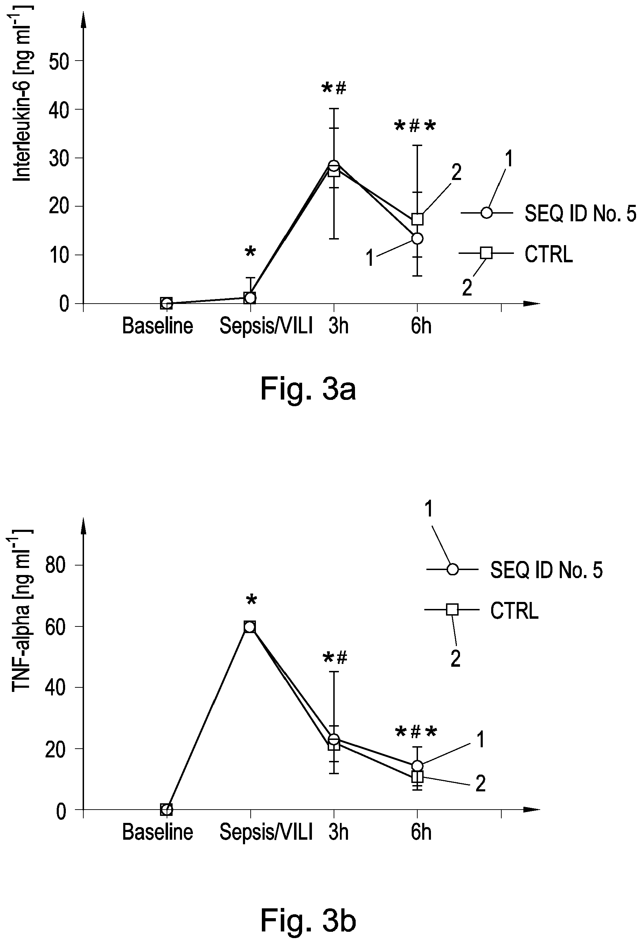

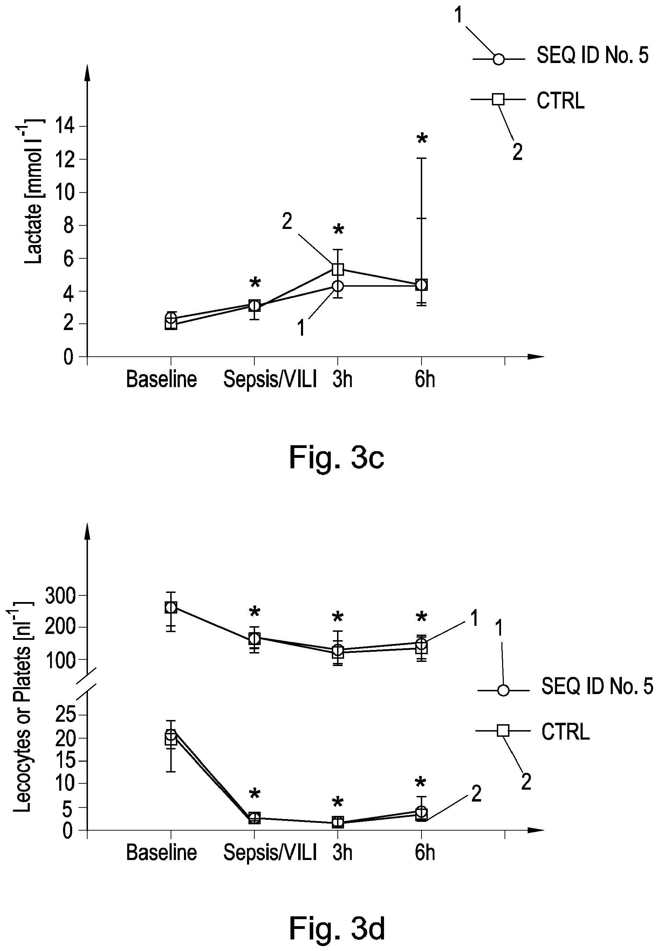

Distribution of histopathological lung injury summarized in Fig. 3. Data of lung regions (each containing periphery and bronchial area) are expressed as median (IQR). * indicates P<0.05 vs. Group (1).

**Table 2 shows the development of alveilar and interstitial edema formation hemorrhage, inflammatory infiltration, epithelial destruction, microacetelectasis and oversdistension in animals treated with a compound of SEQ ID NO:5 versus control (CTRL) animals**

| | non - dependent | | dependent | |
|---|---|---|---|---|
| Parameter | SEQ ID No. 5 | CTRL | SEQ ID No. 5 | CTRL |
| alveolar edema | 0 (0) | 0 (0,2) | 0 (0,1) | 0 (0,4) |
| interstitial edema | 0,9 (1,1) | 1,1 (1,3) | 1,3 (0;8) | 1,0 (0,8) |
| hemorrhage | 0 (0) | 1,1 (1,6)* | 0,1 (0,8) | 0,9 (1,3)* |
| inflammatory infiltration | 3,8 (1,4) | 3,5 (0,9) | 3,4 (1,6) | 3,6 (1,2) |
| epithelial destruction | 0 (0) | 0 (0) | 0 (0) | 0 (0) |
| microatelectasis | 3,8 (1,4) | 3,9 (1,6) | 3,8 (2,4) | 3,5 (2,2) |
| overdistension | 4,1 (0,9) | 3,6 (1,8) | 3,5(1,7) | 3,5 (1,2) |

### Discussion

The key result of present study investigating the influence of SEQ ID:NO 5 peptide-inhalation in a porcine model of LPS-induced lung injury is that SEQ ID:NO 5-peptide significantly reduced intrapulmonary inflammatory response at 6 hours post insult.

### Model characteristics

LPS becomes present as glycolipids of gram-negative bacteria in systemic bacteremia and can trigger inflammatory response to the point of septic shock and cardio-circulatory failure. Systemic effects of LPS in pigs include hemodynamic deterioration along with increased pulmonary arterial pressure and acute leucopenia (Matute-Bello G et al, Am J Physiol Lung Cell Mol Physiol 2008, 295(3):L379-399), which is consistent with findings of this study. Intrapulmonary changes due to LPS infusion include accumulation of leucocytes and alveolar macrophages as well endothelial injury (Wang HM et al, Eur Surg Res 2008, 40(4):305-316). In contrast to other models (i.e. bronchoalveolar lavage), no immediate atelectases and gas exchange impairment are generated by LPS-induced sepsis. In pigs LPS-induced morphologic lung changes in computer tomographic imaging and histopathologic lung damage develop over several hours (Otto CM et al, J Appl Physiol 2008, 104(5):1485-1494). Septic shock and therapy-refractory hemodynamic failure limit the maximum LPS infusion dosages in experimental models. The present model shows a significant worsening of PaO₂/FiO₂ and respiratory mechanics as well as signs of lung injury in the post-mortem analysis.

### Influence on inflammatory response

In response to LPS infusion TNF-α and IL-1β are released into the systemic circulation. In early lung injury alveolar macrophages are the main source of inflammatory cytokines that trigger inflammatory response by e.g. enhancing neutrophil accumulation. (Mittal N, Sanyal SN: Cycloxygenase inhibition enhances the effects of surfactant therapy in endotoxin-induced rat model of ARDS. Inflammation 2011, 34(2):92-98. Matthay MA, Zemans RL: The acute respiratory distress syndrome: pathogenesis and treatment. Annu Rev Pathol 2011, 6:147-163). In the present test system a high circulating plasma levels of TNF-α and IL-6 with a peak within three hours following sepsis induction was detected. Pathophysiological relevance is supported by data demonstrating that early and high circulating levels of IL-6 are associated with increased mortality. Interestingly, repetitive SEQ ID NO:5-peptide inhalation significantly attenuated pulmonary expression of key inflammatory markers like TNF-α, IL-6, and COX-2. Plasma levels of TNF-α and IL-6 were less affected. In the present study, inflammatory marker genes were detected directly in lung tissue. The level of expression was not dependent on the localization within the lung, which can be attributed to the systemic character of LPS infusion.

Tenascin-c, an extracellular matrix glycoprotein, is particularly involved in early inflammatory phase and is induced by inflammatory cytokines, lung remodeling, and fibroproliferation. (Chiquet-Ehrismann R, Chiquet M: Tenascins: regulation and putative functions during pathological stress. The Journal of pathology 2003, 200(4):488-499. Snyder JC, Zemke AC, Stripp BR: Reparative capacity of airway epithelium impacts deposition and remodeling of extracellular matrix. American journal of respiratory cell and molecular biology 2009, 40(6):633-642.) Tenascin-c was significantly lower in the Groups (1), suggesting that SEQ ID NO:5-peptide inhalation mitigates the activity associated with inflammation.

### Conclusion

In a porcine model of systemic inflammatory response related lung injury a repetitive inhalation of a SEQ ID NO:5-peptide significantly attenuated the intrapulmonary expression of inflammatory marker genes.

Inhalation of a compound of the present invention represent a novel option to attenuate inflammatory response. Inhalation of the SEQ ID NO:5-peptide mitigates the intrapulmonary expression of key inflammatory mediators in early sepsis-induced lung injury in pigs.

### Description of the Figures:

Fig. 1 schematically summarizes the experimental protocol for measuring pulmonary inflammation and effects of administration of a compound of the present invention.
Fig. 2a, 2b show the decrease of the quotient of arterial partial pressure of oxygen and FiO₂ (PaO₂/FiO₂) after sepsis and ventilation (Fig. 2a), and the decrease of dynamic lung compliance (C_{dyn}) within 3 hours after administration of a compound of SEQ ID:NO 5 (curve 1) and control (CTRL, curve 2) which both persisted without recovery after 3 hours (Fig. 2b).
Figures 3a to 3d show the increase of plasma levels of IL-6 (Fig. 3a) and TNF-alpha (Fig. 3b), rising lactate levels (Fig. 3c) and decreases in platelet count (Fig. 3d) within three hours after LPS infusion.

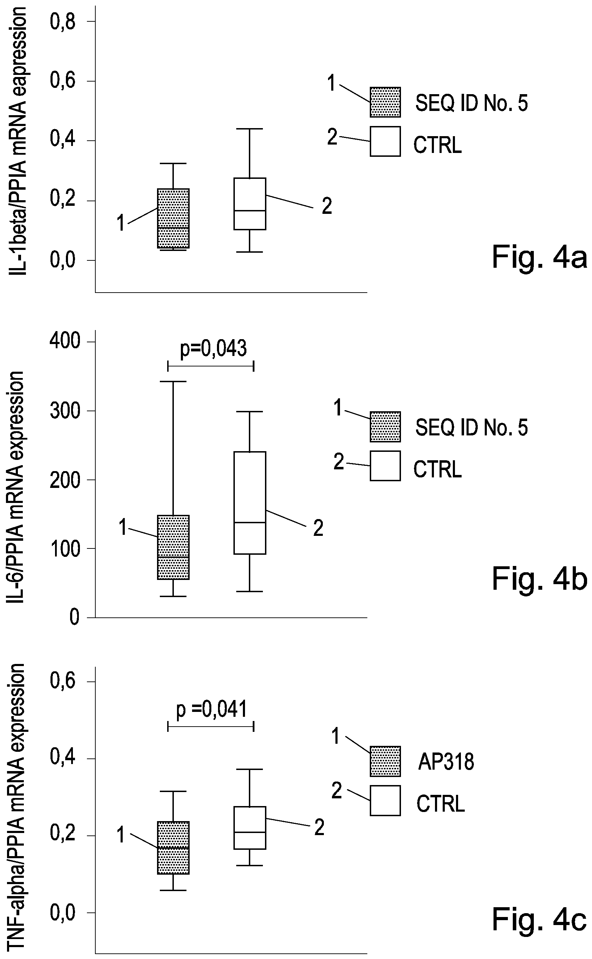

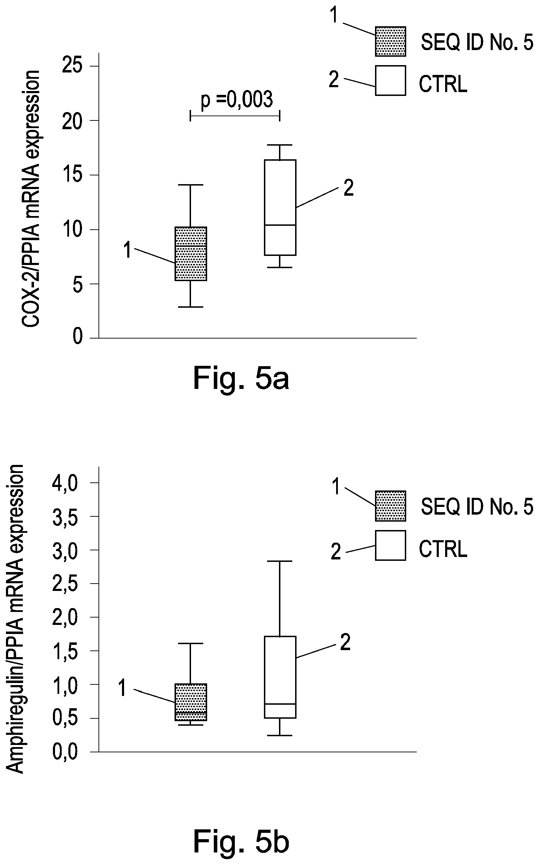

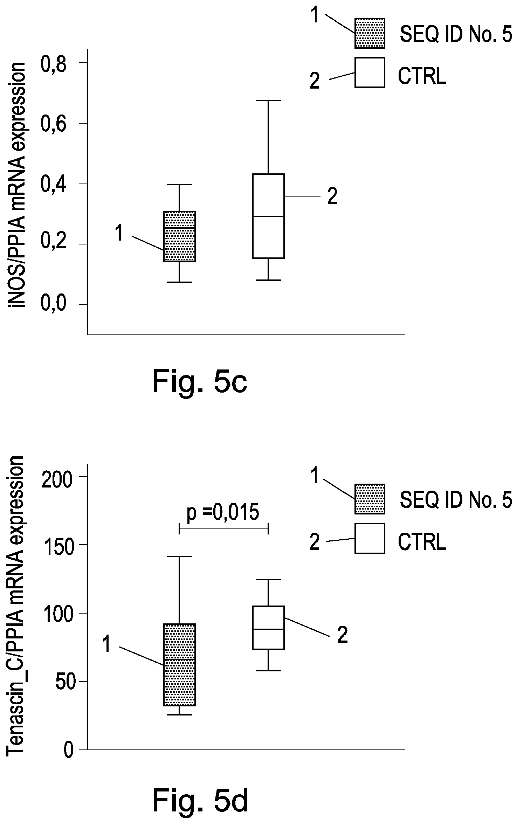

Figures 4a to 5d show intrapulmonary mRNA quantification of the expression of IL-1β (Fig. 4a), IL-6 (Fig. 4b), TNF-α (Fig. 4c), COX-2 (Fig. 5a), amphiregulin (Fig. 5b), INOS (Fig. 5c) and Tenascin (Fig. 5d) following inhalation of a compound of SEQ ID NO:5 (each curve 1 in all figures) beside control administration (CTRL, each curve 2 in all figures).
Fig. 6a to 6c show results of post-mortem macroscopic and histologic evaluations regarding lung injuries (Global lung injury in Fig. 6a, Hemorrhage/Congestion Score in Fig. 6b and Pulmonary wet/dry ratio in Fig. 6c) of animals treated with a compound of SEQ ID NO:5 (Group 1) versus control treated animals (Group 2).

## Claims

1. A cyclized compound of the amino acid sequence of formula I of SEQ ID NO:5:
Cyclo(4-aminobutanoic acid-GQRETPEGAEAKPWYD),
wherein an amide bond is formed between the amino group of the N-terminal 4-aminobutanoic acid residue and the side chain carboxyl group attached to the β-carbon of the C-terminal aspartic acid residue
for use in the treatment of intrapulmonary inflammation, sepsis, systemic and organ inflammation by repetitive inhalation.

2. A compound for use according to claim 1 or claim 1, wherein the cyclized compound of formula I is in the form of a salt.

3. A compound for use according to claim 2, wherein the salt is a hydrochloride.

## Patentansprüche

1. Cyclisierte Verbindung der Aminosäuresequenz der Formel I von SEQ ID Nr. 5:
Cyclo(4-aminobutansäure-GQRETPEGAEAKPWYD),
worin eine Amidbindung zwischen der Aminogruppe des N-terminalen 4-aminobutansäurerests und der am β-Kohlenstoff des C-terminalen Asparaginsäurerests angebrachten Seitenketten-Carboxylgruppe gebildet ist,
zur Verwendung bei der Behandlung von intrapulmonaler Entzündung, Sepsis, systemischer Entzündung und Organentzündung durch wiederholte Inhalation.

2. Verbindung zur Verwendung gemäß Anspruch 1 oder Anspruch 1, wobei die cyclisierte Verbindung der Formel I in Form eines Salzes vorliegt.

3. Verbindung zur Verwendung gemäß Anspruch 2, wobei das Salz ein Hydrochlorid ist.

## Revendications

1. Composé cyclisé de la séquence d'acides aminés de formule 1 de SEQ ID NO:5 :
Cyclo(acide4-aminobutanoïque-GQRETPEGAEAKPWYD),
dans lequel une liaison amide est formée entre le groupe amino du résidu d'acide4-aminobutanoïque N-terminal et le groupe carboxyle à chaîne latérale attaché au carbone β du résidu d'acide aspartique C-terminal, à utiliser dans le traitement d'une inflammation intrapulmonaire, d'une septicémie, d'une inflammation systémique et d'organe par inhalation répétitive.

2. Composé à utiliser selon la revendication 1 ou la revendication 1, dans lequel le composé cyclisé de formule 1 est sous la forme d'un sel.

3. Composé à utiliser selon la revendication 2, dans lequel le sel est un hydrochlorure.


### SEQUENCE LISTING

<110> APEPTICO Forschung und Entwicklung GmbH
<120> Attenuation of intrapulmonary inflammation
<130> A 18761
<150> A 50158/2014
   <151> 2014-03-04
<160> 9
<170> PatentIn version 3.5
<210> 1
   <211> 17
   <212> PRT
   <213> Artificial Sequence
<220>
   <223> cyclized compound for use in inflammation
<220>
   <221> DISULFID
   <222> (1)..(17)
<400> 1
   Cys Gly Gln Arg Glu Thr Pro Glu Gly Ala Glu Ala Lys Pro Trp Tyr
   1               5                   10                  15
   Cys
<210> 2
   <211> 19
   <212> PRT
   <213> Artificial Sequence
<220>
   <223> cyclized compound for use in inflammation
<220>
   <221> TURN
   <222> (1)..(19)
   <223> amide bond formed between the amino group attached to the epsilon-carbon atom of the N-terminal lysine residue and the side chain carboxyl group attached to the gamma-carbon atom of the C-terminal glutamic acid residue
<400> 2
   Lys Ser Pro Gly Gln Arg Glu Thr Pro Glu Gly Ala Glu Ala Lys Pro
   1               5                   10                  15
   Trp Tyr Glu
<210> 3
   <211> 17
   <212> PRT
   <213> Artificial Sequence
<220>
   <223> cyclized compound for use in inflammation
<220>
   <221> TURN
   <222> (1)..(17)
   <223> amide bond formed between the amino group attached to the epsilon-carbon atom of the side chain of the N-terminal lysine residue and the carboxyl group of the C-terminal glycine residue
<400> 3
   Lys Gly Gln Arg Glu Thr Pro Glu Gly Ala Glu Ala Lys Pro Trp Tyr
   1               5                   10                  15
   Gly
<210> 4
   <211> 17
   <212> PRT
   <213> Artificial Sequence
<220>
   <223> cyclized compound for use in inflammation
<220>
   <221> TURN
   <222> (1)..(17)
   <223> amide bond formed between the amino group attached to the delta-carbon of the side chain of the N-terminal ornithine residue and the carboxyl group of the C-terminal glycine residue
<220>
   <221> MOD_RES
   <222> (1)..(1)
   <223> X = ornithine
<400> 4
   Xaa Gly Gln Arg Glu Thr Pro Glu Gly Ala Glu Ala Lys Pro Trp Tyr
   1               5                   10                  15
   Gly
<210> 5
   <211> 17
   <212> PRT
   <213> Artificial Sequence
<220>
   <223> cyclized compound for use in inflammation
<220>
   <221> MOD_RES
   <222> (1)..(1)
   <223> X = 4-aminobutanoic acid
<220>
   <221> TURN
   <222> (1)..(17)
   <223> amide bond formed between the amino group of the N-terminal 4-aminobutanoic acid residue and the side chain carboxyl group attached to the beta-carbon of the C-terminal aspartic acid residue
<400> 5
   Xaa Gly Gln Arg Glu Thr Pro Glu Gly Ala Glu Ala Lys Pro Trp Tyr
   1               5                   10                  15
   Asp
<210> 6
   <211> 17
   <212> PRT
   <213> Artificial Sequence
<220>
   <223> cyclized compound for use in inflammation
<220>
   <221> MOD_RES
   <222> (1)..(1)
   <223> X = beta-alanine
<220>
   <221> TURN
   <222> (1)..(17)
   <223> amide bond formed between the amino group of the N-terminal β-alanine (3-aminopropanoic acid) residue and the side chain carboxyl group attached to the gamma-carbon of the C-terminal glutamic acid residue
<400> 6
   Xaa Gly Gln Arg Glu Thr Pro Glu Gly Ala Glu Ala Lys Pro Trp Tyr
   1               5                   10                  15
   Glu
<210> 7
   <211> 16
   <212> PRT
   <213> Artificial Sequence
<220>
   <223> cyclized compound for use in inflammation
<220>
   <221> TURN
   <222> (1)..(16)
   <223> amide bond between N of amino group of the N-terminal glycine and carbon C1 of carboxyl group of 7-amino-heptanoic acid, and amide bond between N of amino group of 7-amino-heptanoic acid and C of carboxyl group of the C-terminal tyrosine
<220>
   <221> MOD_RES
   <222> (1)..(1)
   <223> X = 7-amino-heptanoic acid
<400> 7
   Xaa Gly Gln Arg Glu Thr Pro Glu Gly Ala Glu Ala Lys Pro Trp Tyr
   1               5                   10                  15
<210> 8
   <211> 17
   <212> PRT
   <213> Artificial Sequence
<220>
   <223> cyclized compound for use in inflammation
<220>
   <221> MOD_RES
   <222> (1)..(1)
   <223> X = 6-amino-hexanoic acid
<220>
   <221> TURN
   <222> (1)..(17)
   <223> amide bond between N of amino group of the N-terminal glycine and C1 of carboxyl group of the 6-amino-hexanoic acid, and amide bond between N of amino group of the 6-amino-hexanoic acid and C of the carboxyl group of the C-terminal glycine
<400> 8
   Xaa Gly Gln Arg Glu Thr Pro Glu Gly Ala Glu Ala Lys Pro Trp Tyr
   1               5                   10                  15
   Gly
<210> 9
   <211> 17
   <212> PRT
   <213> Artificial Sequence
<220>
   <223> cyclized compound for use in inflammation
<220>
   <221> MOD_RES
   <222> (1)..(1)
   <223> X = amino acid/amino acid sequence with 1 to 4, in particular 1 to 3 members, comprising natural or unnatural amino acids, in particular selected from the amino acid/ amino acid sequence C, KSP, K, ornithin, 4-amino butanoic acid, beta-alanine
<220>
   <221> MOD_RES
   <222> (17)..(17)
   <223> X = one amino acid selected from natural amino acids, in particular selected from the group C, D, G and E
<400> 9
   Xaa Gly Gln Arg Glu Thr Pro Glu Gly Ala Glu Ala Lys Pro Trp Tyr
   1               5                   10                  15
   Xaa